# EUROPEAN PATENT SPECIFICATION

(11) **EP 0 440 992 B1**
(45) Date of publication and mention of the grant of the patent: **14.06.1995**
(21) Application number: 90125848.3
(22) Date of filing: 31.12.1990
(51) Int. Cl.: A61M 25/00

(54) **Reinforced multiple-lumen catheter**
Verstärkter Katheter mit mehreren Lumen
Cathéter renforcé à canaux multiples

(30) Priority: 10.01.1990 US 463285
(43) Date of publication of application: 14.08.1991
(60) Divisional application: 93100896.5
(73) Proprietor: Mahurkar, Sakharam Dhundiraj, Chicago Illinois 60660 (US); Mahurkar, Smriti S., Chicago, Illinois 60660 (US)
(72) Inventor: Mahurkar, Sakharam Dhundiraj, Chicago Illinois 60660 (US); Mahurkar, Smriti S., Chicago, Illinois 60660 (US)
(74) Representative: Grünecker, Kinkeldey, Stockmair & Schwanhäusser Anwaltssozietät

(56) References cited:
- WO-A-84/04664
- FR-A- 2 530 958
- US-A- 4 547 193
- US-A- 4 619 643

## Description

The present invention relates generally to dual-lumen catheters for use in medical applications such as hemodialysis where fluids must flow simultaneously to and from a Patient. This invention relates particularly to such catheters which are intended to remain inserted in patients for prolonged periods of treatment, such as a month or more.

A dual lumen hemodialysis catheter is known from the US-A-4619 643. The dual lumen catheter described therein comprises a reinforced septum dividing the interior of the catheter tube into two lumens. The reinforcement of the septum is provided by two wires extending longitudinally through the septum.

Dual-lumen catheters have come into widespread use for extracorporeal blood purification procedures such as hemodialysis. Blood is withdrawn from the patient through one of the lumens of the catheter and supplied to a hemodialysis unit where the blood is purified, and the resulting purified blood is then returned to the patient through the other lumen of the catheter. Examples of such catheters are shown in U.S. Patents 4,134,402; 4,583,968; 4,568,329 and 4,692,141.

At the present time most dual-lumen catheters used for hemodialysis are made of either polyurethane or silicone rubber. The polyurethane catheters are sufficiently rigid that they can be introduced into a patient's vein percutaneously, without surgery, but such catheters tend to be incompatible with by the human body when left in place for long periods of time (e.g., a month or more). The silicone catheters can be left in place indefinitely without allergic reactions or traumatic problems in most patients, but the initial insertion of such catheters usually requires surgical intervention; the soft, pliable, elastic properties of the silicone which contribute to its compatibility with the human body are the same properties that make it difficult or impossible to insert such catheters percutaneously into a patient's vein.

### Summary Of The Invention

It is a primary object of this invention to provide an improved dual-lumen catheter which can be made of silicone or other relatively soft, elastic materials which are unlikely to be rejected by the body, and thus can be used for long-term applications, and yet can be inserted into a patient without surgery.

It is another important object of this invention to provide an improved dual-lumen catheter which can be made of silicone, and yet can be inserted with the use of a needle, guide wire and peel-apart sheath, i.e., without surgical intervention.

A further object of this invention is to provide an improved dual-lumen catheter which can be adapted for long-term use in femoral veins with little or no danger of infection. In this connection, a related object of the invention is to provide such an improved catheter which permits the access site for the catheter to be located above the thighs of the patient.

A still further object of the invention is to provide an improved multiple lumen catheter which resists kinking, even when bent at angles of less than 90°.

In accordance with the present invention, the foregoing objectives are realized by providing a dual-lumen catheter comprising
an elongated cylindrical tube made of a uniformly soft, elastic material which is compatible with the human body, said tube having an internal planar septum extending along the length thereof to form a pair of longitudinal lumens having distal openings which are offset from each other in the axial direction, and
a diametral reinforcing member extending along the length of said septum, and made of a material which is substantially stiffer than the material of said tube so that the catheter can be advanced longitudinally against a resistance by the application of force proximally of the resistance said reinforcing member being completely embedded within said soft compatible material of said tube and septum so that said stiff reinforcing material is not exposed to blood passing through said lumens,
wherein the reinforcing member
extends substantially across the width of the septum so that said lumens will not collapse by deflection of said septum when a pressure differential of a dialysis system exists across said septum.

### Brief Description Of The Drawings

Other objects and advantages of the invention will become apparent upon reading the following detailed description and upon reference to the accompanying drawings, in which:
FIG. 1 is a perspective view of a dual-lumen hemodialysis catheter assembly embodying the present invention;
FIG. 2 is an enlarged longitudinal section taken along a diameter of the distal portion of the catheter of FIG. 1, perpendicular to the septum inside the catheter, as generally illustrated by line 2-2 in FIG. 1;
FIG. 3 is an end elevation taken at the distal end of the catheter portion shown in FIG. 2, as illustrated by line 3-3 in FIG. 2;
FIG. 4 is a section taken generally along line 4-4 in FIG. 2;
FIG. 5 is a section taken generally along line 5-5 in FIG. 2;
FIG. 6 is a section taken generally along line 6-6 in FIG. 2;
FIG. 7 is an enlarged horizontal section taken through the center of the y-shaped hub of the catheter of FIG. 1;
FIG. 8 is a section taken generally along line 8-8 in FIG. 7;
FIG. 9 is a section taken generally along line 9-9 in FIG. 7;
FIG. 10 is a section taken generally along line 10-10 in FIG. 7;
FIG. 11 is a fragmentary longitudinal section through the main body portion of a dual lumen catheter, normal to the septum, containing a modified reinforcing member;
FIG. 12 is a section taken generally along line 12-12 in FIG. 11;
FIG. 13 is a perspective view of a modified dual-lumen hemodialysis catheter assembly embodying the invention;
FIG. 14 is an enlarged longitudinal section taken generally along line 14-14 in FIG. 13;
FIG. 15 is an end elevation taken at the distal end of the catheter portion shown in FIG. 14, as illustrated by line 15-15 in FIG. 14;
FIG. 16 is a section taken generally along line 16-16 in FIG. 14;
FIG. 17 is a section taken generally along line 17-17 in FIG. 14;
FIG. 18 is a section taken generally along line 18-18 in FIG. 14;
FIG. 19 is a longitudinal section similar to FIG. 2 but showing a modified embodiment of the invention;
FIG. 20 is an end elevation taken at the distal end of the catheter portion shown in FIG. 19, as illustrated by line 20-20 in FIG. 19;
FIG. 21 is a section taken generally along line 21-21 in FIG. 19;
FIG. 22 is a section taken generally along line 22-22 in FIG. 19;
FIG. 23 is a section taken generally along line 23-23 in FIG. 19;
FIG. 24 is a longitudinal section similar to FIG. 2 but showing another modified embodiment of the invention;
FIG. 25 is an end elevation taken at the distal end of the catheter portion shown in FIG. 24, as illustrated by line 25-25 in FIG. 24;
FIG. 26 is a section taken generally along line 26-26 in FIG. 24;
FIG. 27 is a section taken generally along line 27-27 in FIG. 24;
FIG. 28 is a section taken generally along line 28-28 in FIG. 24;
FIG. 29 is a longitudinal section similar to FIG. 2 but showing a further modified embodiment of the invention;
FIG. 30 is an end elevation taken at the distal end of the catheter portion shown in FIG. 29, as illustrated by line 30-30 in FIG. 29;
FIG. 31 is a section taken generally along line 31-31 in FIG. 29;
FIG. 32 is a section taken generally along line 32-32 in FIG. 29;
FIG. 33 is a section taken generally along line 33-33 in FIG. 29;
FIG. 34 is a front elevation of the abdominal portion of a patient having the catheter of FIG. 1 implanted in a femoral vein via a subcutaneous tunnel;
FIG. 35 is an enlarged section illustrating the cutaneous tunnel in the patient of FIG. 34 with a guide-wire, catheter and peel-apart sheath inserted therethrough;
FIG. 36 is an enlarged section illustrating a catheter extending through the cutaneous tunnel, and then through a second peel-apart sheath extending into the femoral vein, in the patient of FIG. 34;
FIG. 37 is an enlarged section illustrating the catheter implanted in the femoral vein of the patient of FIG. 34;

While the invention is susceptible to various modifications and alternative forms, specific embodiments thereof have been shown by way of example in the drawings and will herein be described in detail. It should be understood, however, that it is not intended to limit the invention to the particular forms disclosed, but on the contrary, the intention is to cover all modifications, equivalents, and alternatives falling within the scope of the invention as defined by the appended claims.

### Description Of The Preferred Embodiments

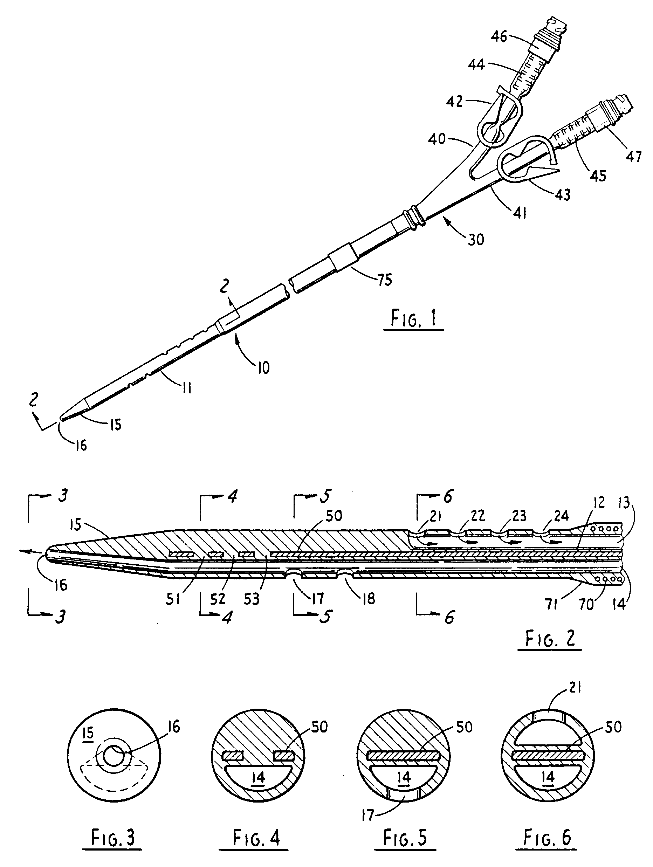

Turning now to the drawings and referring first to FIGS. 1-6, there is shown a dual-lumen hemodialysis catheter 10 of the general type described in Mahurkar U.S. Patent No. 4,583,968, issued April 22, 1986, for "smooth Bore Double Lumen Catheter". This catheter 10 has a cylindrical body portion 11 made of silicone. The body portion 11 is hollow except for a flat, longitudinal, diametral septum 12 which divides the interior of the hollow cylinder into two parallel lumens 13 and 14, each having a D-shaped cross section (FIGS. 2 and 6). As illustrated by the arrows in FIG. 2, the lumen 13 is the blood-intake lumen, and the lumen 14 is the blood-return lumen.

At the distal end of the catheter, the exterior surface of the cylinder 11 merges into a smoothly tapered conical tip 15. On the inside, the blood return lumen 14 extends longitudinally all the way through the tip 15, bending slightly as it passes through the tip so that it opens at 16 near the center of the distal end of the conical tip, as can be seen in FIGS. 2 and 3. Within the tip 15, the cross-sectional shape of the lumen 14 gradually changes from D-shaped at the proximal end of the tip 15 (see FIG. 4) to circular at the distal end of the tip (see FIG. 3). The transition from D to circular is illustrated by the broken lines in FIG. 3.

In addition to the opening 16 at the distal end of the blood-return lumen 14, a pair of apertures 17 and 18 are formed in the side wall of the return lumen. These apertures 17 and 18, which are spaced longitudinally away from the distal opening 16 toward the proximal end of the catheter, ensure the flow of blood through the return lumen 14 even in situations where the distal opening 16 might become wholly or partially blocked. The area of the apertures 16, 17 and 18 is preferably at least equal to the transverse cross-sectional area of the return lumen 14.

In order to provide a longitudinal spacing between the distal openings of the two lumens 13 and 14, the blood-intake lumen 13 is terminated at an opening 21 in the side wall of the catheter. Additional openings 22-24 spaced longitudinally from the opening 21 permit blood to enter the lumen 13 freely without excessive vacuum in the event of a blockage of the opening 21 against the wall of the vein into which the catheter 10 is inserted.

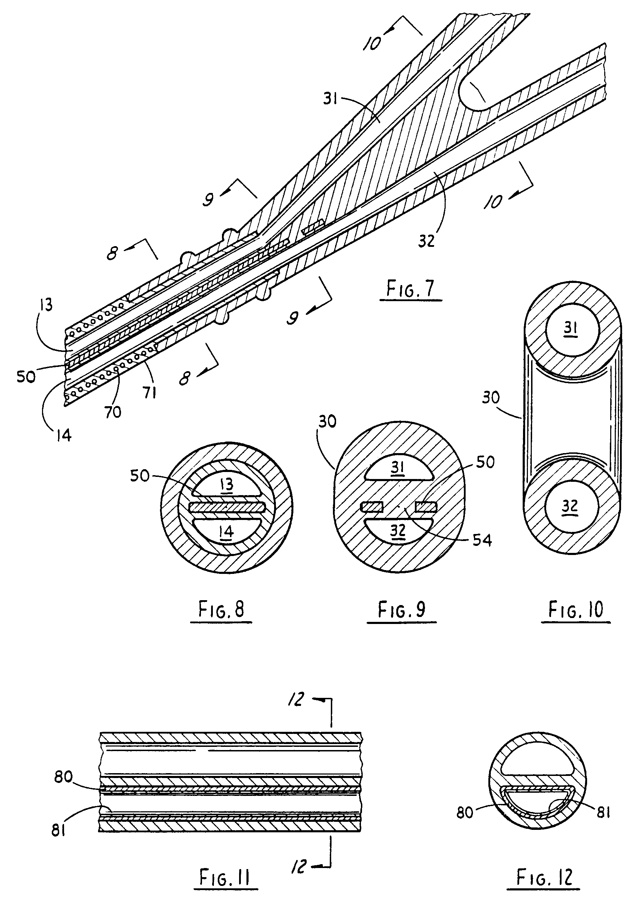

At the proximal end of the catheter 10, the two D-shaped lumens 13 and 14 open into a Y-shaped connector or hub 30 which forms two internal passageways 31 and 32 (see FIGS. 7-10) communicating with the proximal ends of the catheter lumens. As can be seen in FIGS. 7 and 9, the distal ends of the hub passageways 31 and 32 are D-shaped so that they form extensions of the catheter lumens 13 and 14, respectively. The passageways 31 and 32 diverge from each other and assume a circular cross section (see FIG. 10) as they extend toward the proximal end of the hub, and they also increase in cross-sectional area, as can be seen in FIGS. 7 and 10. The hub 30 is preferably molded in place on the end of the catheter, using mold inserts to form the hub passageways 31 and 32. Alternatively, the walls of the catheter lumens may be expanded at the proximal end of the catheter to fit over the corresponding portions of a preformed hub 30 with the inside walls of the catheter lumens being bonded to the mating walls of the hub 30.

To facilitate connection of the catheter hub 30 to the conventional tubes leading to a dialysis unit, and also to accommodate a pair of clamps for opening and closing the blood intake and return passageways the hub 30 forms a pair of extension tubes 40 and 41 (FIG. 1). These extension tubes 40 and 41 are long enough to receive a pair of conventional clamps 42 and 43 for opening and closing the respective tubes. The extension tubes 40 and 41 are relatively soft and flexible, so that they can be easily manipulated and also easily closed by the pressure of the clamps 42 and 43. The clamps 42 and 43 serve as on-off valves for controlling the flow of blood between the catheter and the dialysis unit. At the proximal end of the hub 30, the hub passageways 31 and 32 open onto a pair of ferrules 44 and 45 formed as integral parts of luer connectors 46 and 47. The luer connectors serve as coupling means for coupling the proximal ends of the extension tubes to the flexible tubes leading to the extracorporeal blood treatment unit.

In accordance with one aspect of the present invention, a reinforcing member is embedded in the septum 12 and extends along the full length of the intake lumen 13. The reinforcing member is made of a material which is substantially stiffer than the silicone used to form the main body of the catheter, so that the catheter can be advanced against a resistance by the application of force to the proximal end of the catheter. In the illustrative embodiment of FIGS. 1-6, the reinforcing member is in the form of a flat polymeric strip 50 inserted longitudinally within a hollow septum 12. The width of the strip 50 is preferably slightly greater than the inside diameter of the silicone tube so that the strip extends slightly into the cylindrical walls of the tube.

The reinforcing strip 50 is made of a material that is stiff enough to transmit longitudinally applied forces from the proximal end of the catheter to the conical tip at the distal end of the catheter so that the catheter can be readily inserted into a patient percutaneously, i.e., without surgical intervention. One suitable material for the reinforcing strip is nylon, which provides the requisite degree of stiffness in a strip 3,429 mm (0.135 inch) wide and 0,305 mm (0.012 inch) thick.

With the reinforcing strip, a silicone catheter can be easily inserted through a peel-apart sheath. Such a sheath is typically used after a guide wire has been introduced into a patient's vein by use of an access needle. The puncture formed by the needle is subsequently dilated by a smooth plastic tip on the end of a dilator telescoped through the pull-apart sheath and inserted a short distance into the vein. The dilator is then removed, the catheter is inserted through the sheath, and finally the sheath is removed by stripping it apart along two longitudinal tear lines.

Without the reinforcing member provided by this invention, attempts to insert a silicone catheter by the technique described above have usually resulted in radial expansion of the catheter at the entry to the sheath, due to the frictional resistance of the silicone surface engaging the inside wall of the sheath. Rather than causing the catheter to slide through the sheath, the applied insertion force resulted in expansion of the soft, elastic silicone material of the catheter body against the proximal end of the sheath, thereby preventing the catheter from sliding through the sheath.

With the present invention, however, the insertion force applied to the proximal end of the catheter is transmitted by the reinforcing member to the tip of the catheter, thereby "pulling" the catheter through the sheath. That is, the relatively stiff reinforcing member provides the catheter with sufficient column strength for easy insertion through the sheath. Although presently available materials that have the stiffness needed in the reinforcing material are not suitable for long-term implantation in the human body because of incompatibility problems such as localized allergic reactions, the present invention enables the reinforcing material to be isolated from the body. Thus, in the embodiment of FIG. 1, for example, the nylon reinforcing material is surrounded by the silicone material of the hollow septum. Therefore, it is only the silicone material that comes into contact with internal body tissues or fluids.

In addition, the reinforcing member prevents collapse of one of the lumens due to the pressure differential that normally exists across the septum of a dual-lumen catheter while it is being used to withdraw blood under a negative pressure in one lumen and to return blood under a positive pressure in the other lumen. With a silicone catheter, it is possible for the septum and the outside wall of the negative-pressure lumen to collapse together, thereby closing that lumen. The reinforcing strip, however, provides sufficient rigidity to the septum to prevent such collapsing of the negative-pressure lumen. Even when the outer wall of the lumen is not reinforced, the curvature of that wall normally prevents it from being collapsed against the septum as long as the septum itself cannot be distorted substantially toward the outer wall.

The preferred method of making the catheter of FIG. 1 is to extrude the main body portion of the catheter as a cylindrical tube having a hollow septum dividing the interior of the tube into two D-shaped lumens and a central cavity for receiving the reinforcing strip 50 (see FIG. 6). The extruded tube can be cut into the desired lengths for individual catheters. The strip 50 is then inserted into the central cavity, and the tip 15 and the hub 30 are formed on opposite ends of the tube by conventional techniques. Alternatively, the dual-lumen tube can be co-extruded with a continuous reinforcing strip in the septum.

In the particular embodiment illustrated in FIGs. 1-6, the catheter tip 15 is molded as a unitary part of the catheter tube. This is accomplished by placing a tip mold around the distal end of the tube which forms the two D-shaped lumens, with the reinforcing strip in place and with mold inserts in place for forming the end of the intake lumen 13 and the extension of the return lumen 14 through the tip. As can be seen in FIGS. 2 and 4, the end portion of the reinforcing strip is preferably provided with a plurality of holes 51, 52 and 53 so that during the molding of the tip the molten silicone flows through the holes in the reinforcing strip (the entire distal end portion of the tube within the tip-forming mold is normally melted in this process). Then when the silicone is subsequently solidified, the reinforcing strip is locked firmly to the tip by the material that solidifies within the holes 51-53 of the strip. This interlocking of the reinforcing strip and the silicone holds the reinforcing strip securely in place, and ensures that the strip does not penetrate or separate through the relatively soft silicone tip during and after the insertion of the catheter.

At the proximal end of the catheter, the reinforcing strip 50 is similarly interlocked with the silicone that forms the hub 30. Thus, as can be seen in FIGS. 7 and 9, the proximal end of the strip 50 forms a hole 54 for receiving molten silicone during the in-situ molding of the hub 30. Then when the silicone solidifies, the strip 50 is securely interlocked with the hub 30.

To minimize kinking, the catheter of FIGS. 1-6 has a spiral 70 of relatively stiff material embedded in the cylindrical wall of the catheter along a substantial portion of the length of the catheter. The spiral 70 is preferably a thin metal wire wound helically around the extruded silicone tube, and then covered with a layer 71 of silicone so that the wire is not exposed to body tissue. The silicone is preferably applied as a liquid so that it flows around the spiral 70 and becomes a part of the silicone side walls of the catheter. If desired, a strong polymeric monofilament, e.g., nylon, may be used instead of the metal wire. The spiral 70 will always tend to retain its cylindrical shape, and thus also tends to hold the outer wall of the catheter in a cylindrical shape, thereby resisting kinking of the catheter. Consequently, the catheter can be bent, even at acute angles, without kinking. The use of such anti-kinking spirals in catheters is not new by itself, but the use of this feature in multiple-lumen silicone catheters such as those of the present invention leads to significant advantages in certain applications, as will be described in more detail below.

As shown in FIG. 1, a "Dacron" collar 75 is provided around the main body portion of the catheter to facilitate the growth of body tissues directly around the catheter where it enters the patient's body. The use of such a collar is well known in the catheter art.

FIGS. 11 and 12 illustrate a modified form of reinforcing member 80, which has a hollow D-shaped cross section, rather than the flat strip used in the embodiment of FIGS. 1-10. The D-shaped reinforcing member 80 is dimensioned to fit snugly within the intake lumen 13, with the distal end of the member 80 abutting the solid tip at the distal end of the lumen 13 (see FIG. 2). After the reinforcing member 80 has been inserted into the lumen 13, a thin layer 81 of silicone is formed on the inside walls of the member 80, e.g., by simply wicking a silicone liquid through the interior of the member 80 by capillary action.

If desired, the D-shaped reinforcing member 80 may be inserted into the intake lumen 13 only during insertion of the catheter into the patient. After the catheter is inserted, the reinforcing member 80 can be withdrawn from the catheter via the hub 30.

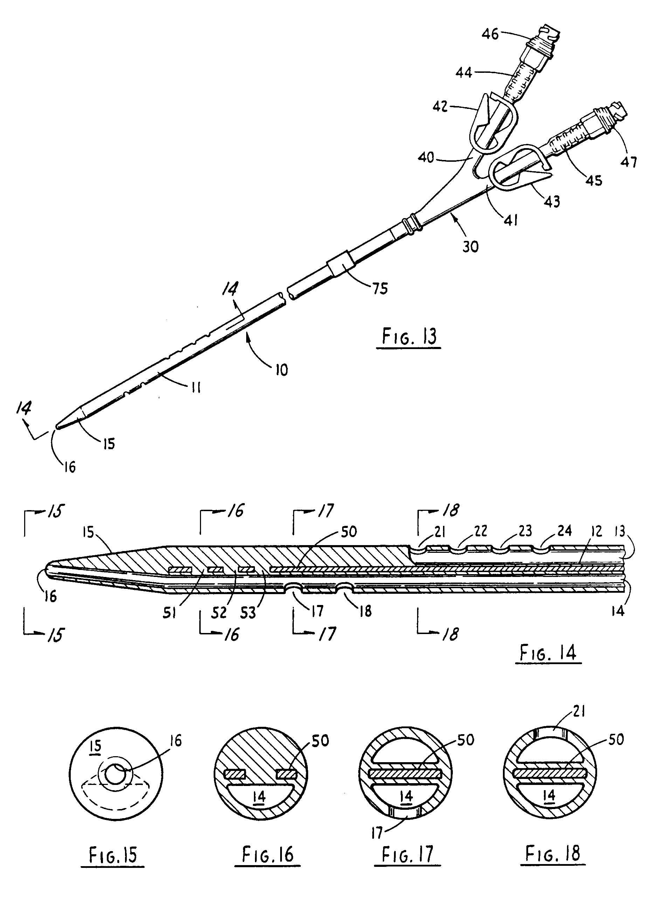

FIGS. 13-18 illustrate a catheter like the catheter of FIGS. 1-10 except that there is no reinforcing spiral around the outside wall of the catheter. This catheter is preferred for applications where kinking is not a problem, such as for implantation in the subclavian vein. The parts of the this catheter which are the same as those of the catheters of FIGS. 1-10 have been identified by the same reference numerals in FIGS. 13-18.

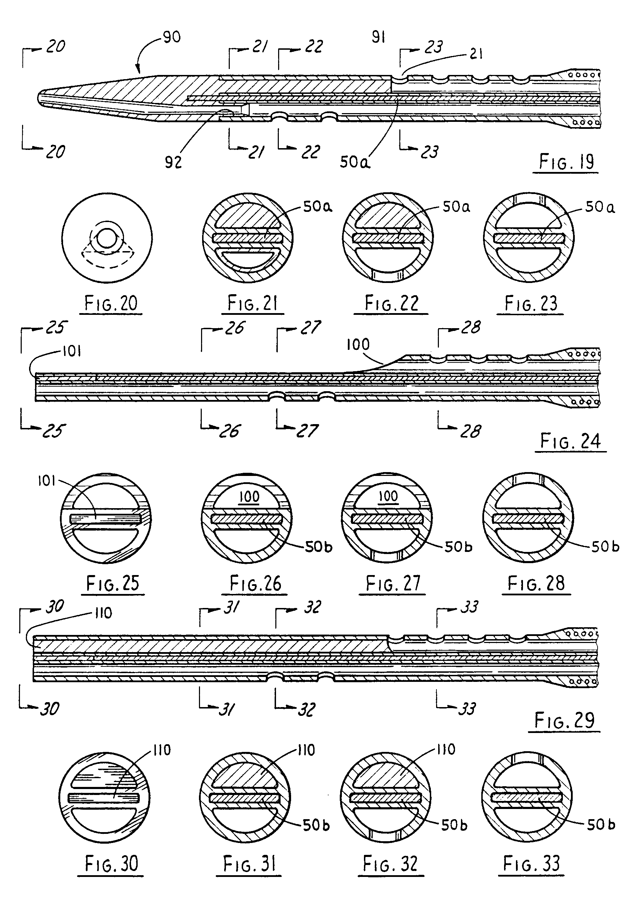

FIGS. 19-33 illustrate different configurations for the distal ends of catheters embodying this invention. Thus, the catheter of FIGS. 19-23 is made with a tapered conical tip 90 which is pre-molded and then inserted into the end of the extruded tube forming the two D-shaped lumens and the hollow septum. The reinforcing strip 50a in this embodiment is not apertured, but it extends slightly beyond the distal end of the cylindrical tube so as to fit into a mating slot formed in the pre-molded tip 90. The tip 90 also forms a D-shaped extension 91 which fits into the intake lumen and extends up to the edge of the first sidewall aperture, so as to form the terminus of the intake lumen. A shorter, hollow extension or "flash" 92 fits into the distal end of the return lumen, and is tapered to provide a smooth transition between the lumen of the cylindrical tube and the continuation of that lumen formed by the hollow tip. This pre-molded tip 90 is preferably made of silicone, and is bonded to the interior walls of the extruded tube by a silicone adhesive.

In the catheter of FIGS. 24-28, the intake lumen is terminated with an open end 100 by simply slicing off the distal portion of the lumen with a beveled cut. The return lumen continues all the way to the distal end of the tube, and retains the same D-shaped cross sectional configuration along its entire length. The reinforcing member 50b terminates in the region between the distal ends of the return lumen and the intake lumen, and the distal end portion of the hollow cavity formed by the septum is filled with a solid plug 101.

In the catheter of FIGS. 29-33, the intake lumen is terminated by an insert 110 which fills the distal end portion of the intake lumen formed by the extruded tube, from the distal end of the tube to the edge of the first sidewall aperture. The return lumen and the reinforcing member are exactly the same as in the catheter of FIGS. 24-28 described above.

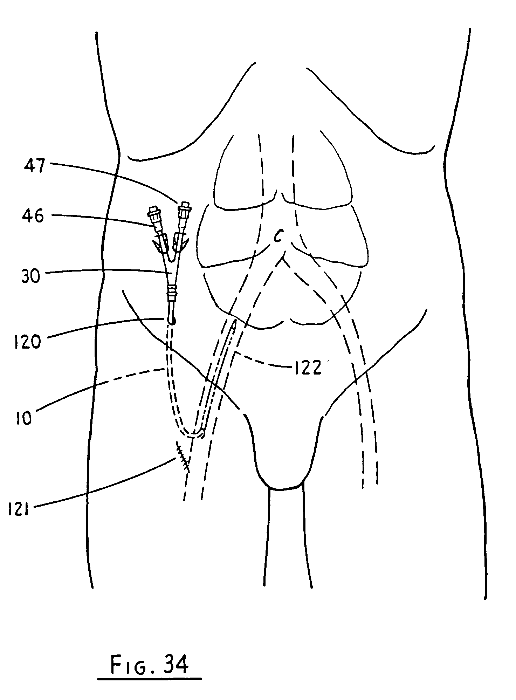

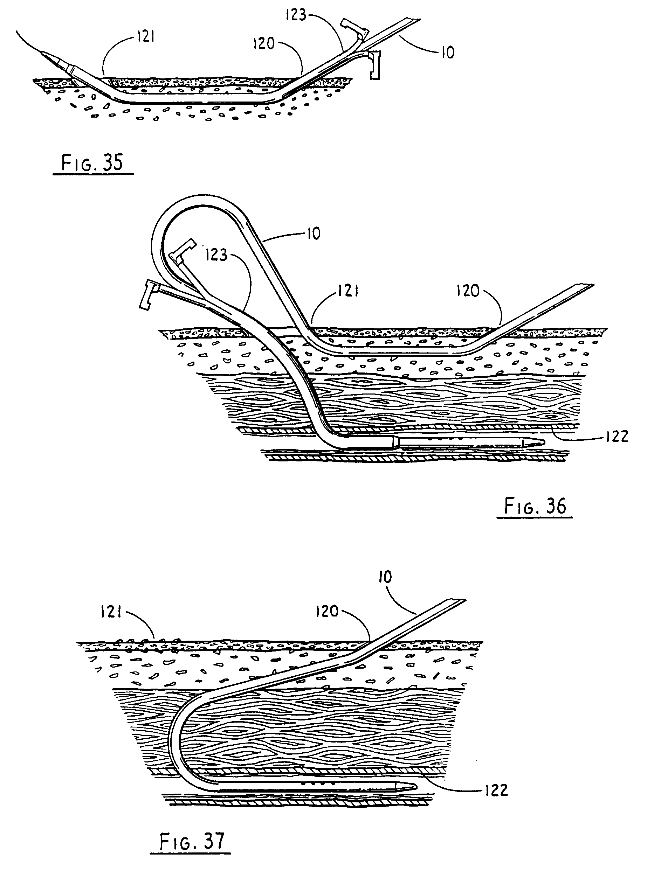

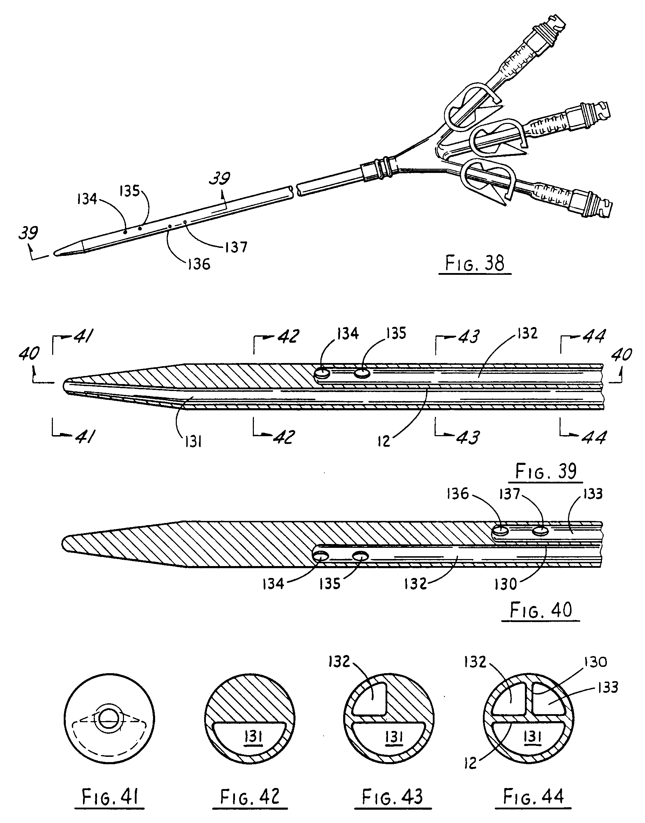

One particularly advantageous application for the kink-resistant catheters of this invention is an improved long-term implantation technique for the femoral vein. In contrast to the subclavian vein, the femoral vein is readily accessible through the thigh and is well removed from critical organs such as the lungs and heart. Nevertheless, the femoral vein has not been a popular access site for hemodialysis catheters because of the higher risk of infection below the inguinal canal. With the kink-resistant catheters provided by the present invention, however, the catheter can extend downwardly from a point above the inguinal ligament through a subcutaneous tunnel, and then bent upwardly for insertion into the femoral vein. This method is illustrated in FIGS. 34-37.

The method for inserting the dual-lumen catheter 10 comprises the following steps: a lateral incision 120 of approximately a 12,7 mm (half inch) is made percutaneously in the anterior abdominal wall. The incision 120 is made approximately 50,8 mm - 76,2 mm (2-3 inches) cephalad to the inguinal ligament. The distal end of the catheter 10 is inserted through the incision 120 by applying pressure on the proximal end of the catheter. The catheter is tunneled through the abdominal wall subcutaneously in a caudal direction toward the thigh. A second incision 121 of approximately 12,7 mm (a half inch) is made approximately 44,45 mm (1.75 inch) below, that is, caudal to, the inguinal ligament. The catheter is pushed out off the body through the second incision 121 in the thigh at a distance of approximately 1.75 inch caudal to the inguinal ligament. In approximately that same region, the fascia is punctured with a percutaneous entry needle. The needle is introduced interiorly through the subcutaneous layers into the femoral vein 122. A flexible guide wire is introduced through the needle and advanced approximately 5-10 cm into the vessel. The needle is then withdrawn. The puncture formed by the needle is dilated using a small plastic tip on the end of a dilator, telescoped inside a peel-apart sheath 123. The dilator is inserted a short distance into the blood vessel and then removed. The dual-lumen catheter 10 which has been extruded from the skin of the thigh, is then threaded onto the guide wire in a cephalad direction and inserted interiorly through the sheath 123 into the femoral vein, in the direction of blood flow within the vein. The sheath 123 is removed by stripping it apart along the two longitudinal tear lines, and the guide wire is pulled out.

The catheter is then advanced in a cephalad direction until the loop protruding above the surface of the thigh is pulled beneath the skin. Both the incisions are then sutured, and the extension tubes of the catheters are connected to the dialysis circuit for hemodialysis.

## Claims

1. A dual-lumen hemodialysis catheter comprising:
an elongated cylindrical tube (10) made of a uniformly soft, elastic material which is compatible with the human body, said tube (10) having an internal planar septum (12) extending along the length thereof to form a pair of longitudinal lumens (13,14), having distal openings (17,18;21,22,23,24) which are offset from each other in the axial direction, and
a diametral reinforcing member (50, 80) extending along the length of said septum (12), and made of a material which is substantially stiffer than the material of said tube (10) so that the catheter can be advanced longitudinally against a resistance by the application of force proximally of the resistance said reinforcing member (50, 80) being completely embedded within said soft compatible material of said tube (10) and septum (12) so that said stiff reinforcing material (50, 80) is not exposed to blood passing through said lumens (13,14), **characterized in that** the reinforcing member (50, 80) extends substantially across the width of the septum so that said lumens (13,14) will not collapse by deflection of said septum (12) when a pressure differential of a dialysis system exists across said septum (12).

2. The dual-lumen catheter of claim 1 wherein said septum (12) is flat so that said lumens (13,14) have D-shaped cross sections.

3. The dual-lumen catheter of claim 1 which includes a hollow conical tip (15) on the distal end of said tube (10), the outside surface of said conical tip (15) merging smoothly with the outside surface of said tube (10), and the inside surface of said conical tip (15) merging smoothly with the inside surface of one of said lumens (14), the distal end of the other of said lumens (13) being longitudinally spaced from the distal end of said tip (15).

4. The dual-lumen catheter of claim 3 wherein said conical tip (15) forms a solid connection from the distal end of said septum (12) and other lumen to the distal end of said tip (15), and the distal end of said reinforcing member (50, 80) engages said solid connection so that forces applied to the proximal end of said tube (10) are transmitted to said tip (15) via said reinforcing member (50, 80).

5. The dual-lumen catheter of claim 3 wherein said septum (12) comprises a pair of transversely spaced longitudinal webs formed as integral parts of said tube (10), and said reinforcing member (50) comprises a flat strip of stiff material inserted longitudinally between said webs and bonded thereto.

6. The dual-lumen catheter of claim 5 wherein the transverse dimension of said reinforcing strip, in the direction parallel to said webs, is greater than the inside diameter of said tube (10) so that said strip extends partially into the walls of the tube (10).

7. The dual-lumen catheter of claim 1 wherein said reinforcing member (80) comprises an elongated tubular member embedded in the wall of one of said lumens (13,14).

8. The dual-lumen catheter of claim 7 wherein both said one lumen (13) and said reinforcing member (80) have D-shaped transverse cross sections.

9. The dual-lumen catheter of claim 7 wherein the inside walls of said reinforcing member (80) are coated with the same material (81) that forms said tube (10) so that said tubular reinforcing member (80) is isolated from body tissues and fluids.

10. The dual-lumen catheter of claim 1 wherein said reinforcing member (50,80) is made of nylon.

11. The dual-lumen catheter of claim 1 wherein said reinforcing member (50,80) and a portion of said tube at the distal end thereof are interlocked.

12. The dual-lumen catheter of one of claims 1 to 11 wherein
said distal end of said strip (50, 80) being connected to the distal end of said tube (10) and having apertures (51,52,53) therein, portions of said tube (10) extending transversely through said apertures (51,52,53) to interlock said strip (50, 80) and said tube (10), said reinforcing member (50, 80) extending along the length of said tube (10) and being made of a material which is substantially stiffer than the material of said tube (10) so that the catheter can be advanced longitudinally against a resistance by the application of force proximally of the resistance and so that said lumen (13,14) will not collapse by deflection of said septum (12) when the pressure differential of a dialysis system exists across said septum (12).

13. The dual-lumen catheter of claim 1 which includes a kink-resisting spiral (70) of reinforcing material wound around a substantial length of said tube (10).

14. The dual-lumen catheter of claim 13 wherein said spiral (70) is embedded in the cylindrical wall of said tube (10).

15. The dual-lumen catheter of claim 3 wherein said conical tip (15) is molded directly onto the end of said tube (10).

16. The dual-lumen catheter of claim 3 wherein the distal end of one of said lumens (13,14) and said septum are connected to said conical tip (15) by solid material.

17. The dual-lumen catheter of claim 3 wherein said conical tip (15) is preformed and bonded to the end of said tube (10), a portion of said preformed tip (15) extending into said tube (10) to form the terminus of one of said lumens (13).

18. The dual-lumen catheter of one of claims 1 to 17 wherein at least one aperture (21,22,23,24) in the side wall of said tube opening into the other of said lumens (13).

19. The multiple-lumen catheter of claim 18 wherein said reinforcing member (50) is removable from said tube (10) so that said reinforcing member (80) can be withdrawn from said tube (10) after the catheter has been inserted into a patient.

## Patentansprüche

1. Hämodialysenkatheter mit Zwei-Lumen, umfassend:
ein längliches Zylinderrohr (10), das aus einem gleichförmig weichen, elastischen Werkstoff hergestellt ist, welcher mit dem menschlichen Körper verträglich ist, wobei das Rohr (10) eine ebene innere Scheidewand (12) aufweist, die sich entlang dessen Länge erstreckt, um ein Paar Längslumen (13, 14) zu formen, die distale Öffnungen (17, 18; 21, 22, 23, 24) aufweisen, welche in axialer Richtung voneinander versetzt angeordnet sind, und
ein diametrales Verstärkungselement (50, 80), das sich entlang der Länge der Scheidewand (12) erstreckt und aus einem Werkstoff hergestellt ist, welcher im wesentlichen steifer ist als der Werkstoff des Rohrs (10), so daß der Katheter gegen einen Widerstand durch Aufbringen einer Kraft nahe des Widerstands längs vorbewegt werden kann, wobei das Verstärkungselement (50, 80) komplett in dem weichen, verträglichen Werkstoff des Rohrs (10) und der Scheidewand (12) eingebettet ist, so daß der steife Verstärkungswerkstoff (50, 80) nicht Blut, daß durch die Lumen (13, 14) hindurchtritt, ausgesetzt ist,
**dadurch gekennzeichnet,**
daß das Verstärkungselement (50, 80) sich im wesentlichen über die Breite der Scheidewand erstreckt, so daß die Lumen (13 14) nicht durch eine Durchbiegung der Scheidewand (12) kollabieren werden, wenn eine Druckdifferenz des Dialysesystems über der Scheidewand (12) vorhanden ist.

2. Der Zwei-Lumenkatheter aus Anspruch 1, worin die Scheidewand (12) flach ist, so daß die Lumen (13, 14) einen D-förmigen Querschnitt aufweisen.

3. Der Zwei-Lumenkatheter aus Anspruch 1, welcher eine hohle konische Spitze (15) an dem distalen Ende des Rohrs (10) umfaßt, wobei die Außenfläche der konischen Spitze (15) glatt in die Außenseite des Rohrs (10) übergeht und die Innenfläche der konischen Spitze (15) glatt in die Innenfläche eines der Lumen (14) übergeht, wobei das distale Ende des anderen der Lumen (13) von dem distalen Ende der Spitze (15) längs beabstandet ist.

4. Der Zwei-Lumenkatheter aus Anspruch 3, worin die konische Spitze (15) eine solide Verbindung von dem distalen Ende der Scheidewand (12) und dem anderen Lumen an dem distalen Ende (15) der Spitze formt, und das distale Ende des Verstärkungselements (50, 80) mit der soliden Verbindung in Eingriff steht, so daß Kräfte, die an dem nahen Ende des Rohrs (10) ausgeübt werden, über das Verstärkungselement (50, 80) auf die Spitze (15) übertragen werden.

5. Der Zwei-Lumenkatheter aus Anspruch 3, worin die Scheidewand (12) ein Paar transversal beabstandete Längsstege als integraler Bestandteil des Rohres (10) aufweist, und das Verstärkungselement (50) einen flachen Streifen eines steifen Materials umfaßt, der längs zwischen die Stege eingeführt und mit diesen verbunden ist.

6. Der Zwei-Lumenkatheter aus Anspruch 5, worin die Querdimension des Verstärkungsstreifens in der Richtung parallel zu den Stegen größer ist als der Innendurchmesser des Rohres (10), so daß der Streifen sich teilweise in die Wände des Rohrs (10) erstreckt.

7. Der Zwei-Lumenkatheter aus Anspruch 1, worin das Verstärkungselement (80) ein längliches Rohrelement umfaßt, daß in der Wand von einem der Lumen (13, 14) eingebettet ist.

8. Der Zwei-Lumenkatheter aus Anspruch 7, worin das Lumen (13) und das Verstärkungselement (80) einen D-förmigen Querschnitt aufweisen.

9. Der Zwei-Lumenkatheter aus Anspruch 7, worin die Innenwände des Verstärkungselementes (80) mit dem selben Werkstoff (81) beschichtet sind, der das Rohr (10) formt, so daß das rohrförmige Verstärkungselement (80) von dem Körpergewebe und den - flüssigkeiten isoliert ist.

10. Der Zwei-Lumenkatheter aus Anspruch 1, worin das Verstärkungselement (50, 80) aus Nylon hergestellt ist.

11. Der Zwei-Lumenkatheter aus Anspruch 1, worin das Verstärkungselement (50, 80) und ein Bereich des Rohrs an dessen distalen Ende ineinandergreifen.

12. Der Zwei-Lumenkatheter aus einem der Ansprüche 1 bis 11, worin das distale Ende des Streifens (50, 80) mit dem distalen Ende des Rohrs (10) verbunden ist und Öffnungen (51, 52, 53) darin aufweist, wobei Bereiche des Rohrs (10) sich transversal durch die Öffnungen (51, 52, 53) erstrecken, um mit dem Streifen (50, 80) und dem Rohr 810) ineinanderzugreifen, wobei das Verstärkungselement (50, 80) sich entlang der Länge des Rohrs (10) erstreckt und aus einem Werkstoff hergestellt ist, welcher steifer ist als der Werkstoff des Rohrs (10), so daß der Katheter gegen einen Widerstand durch aufbringen einer Kraft nahe des Widerstands längs bewegt werden kann und das die Lumen (13, 14) nicht durch Durchbiegung der Scheidewand (12) kollabieren werden, wenn eine Druckdifferenz des Dialysesystems über die Scheidewand (12) vorhanden ist.

13. Der Zwei-Lumenkatheter aus Anspruch 1, welcher eine knickstabile Spirale (70) aus Verstärkungsmaterial umfaßt, die um eine wesentliche Länge des Rohres (10) gewunden ist.

14. Der Zwei-Lumenkatheter aus Anspruch 13, worin die Spirale (70) in der zylindrischen Wand des Rohrs (10) eingebettet ist.

15. Der Zwei-Lumenkatheter aus Anspruch 3, worin die konische Spitze (15) direkt an das Ende des Rohres (10) angeformt ist.

16. Der Zwei-Lumenkatheter aus Anspruch 3, worin das distale Ende eines der Lumen (13, 14) und die Scheidewand mit der konischen Spitze (15) durch einen festen Werkstoff verbunden sind.

17. Der Zwei-Lumenkatheter aus Anspruch 3, worin die konische Spitze (15) vorgeformt und mit dem Ende des Rohrs (10) verbunden ist, wobei ein Bereich der vorgeformten Spitze (15) sich in das Rohr (10) erstreckt, um den Endpunkt von einem der Lumen (13) zu bilden.

18. Der Zwei-Lumenkatheter aus einem der Ansprüche 1 bis 17, worin mindestens eine Öffnung (21, 22, 23, 24) in der Seitenwand des Rohrs zu dem anderen Lumen (13) hin geöffnet ist.

19. Der Zwei-Lumenkatheter aus Anspruch 18, worin das Verstärkungselement (50) von dem Rohr (10) entfernbar ist, so daß das Verstärkungselement (80) aus dem Rohr (10) nach dem der Katheter in einen Patienten eingeführt worden ist, entfernt werden kann.

## Revendications

1. Cathéter pour hémodialyse à canal double comportant :
un tube cylindrique allongé (10) constitué par un matériau élastique uniformément mou, compatible avec le corps humain, ledit tube (10) comprenant un septum plan interne (12) qui s'étend sur la longueur du tube pour constituer une paire de canaux longitudinaux (13, 14) ayant des ouvertures distales (17, 18 ; 21, 22, 23, 24) déportées les unes par rapport aux autres dans la direction axiale, et
un élément de renforcement diamétral (50, 80) qui s'étend sur la longueur dudit septum (12) constitué par un matériau essentiellement plus rigide que le matériau qui constitue ledit tube (10) de telle sorte que l'on peut faire avancer le cathéter longitudinalement lorsqu'on lui oppose une résistance, par l'application d'un effort proximal par rapport à la résistance, ledit élément de renforcement (50, 80) étant complètement enrobé à l'intérieur dudit matériau compatible mou qui constitue ledit tube (10) et du septum (12) de telle sorte que ledit matériau de renforcement rigide (50, 80) n'est pas exposé au sang qui circule à travers lesdits canaux (13, 14), caractérisé en ce que l'élément de renforcement (50, 80) s'étend sensiblement à travers la largeur du septum de telle sorte que lesdits canaux (13, 14) ne s'affaissent pas par la déformation dudit septum (12) lorsqu'une pression différentielle du système de dialyse s'établit à travers ledit septum (12).

2. Cathéter à canal double selon la revendication 1, dans lequel ledit septum (12) est plat de telle sorte que lesdits canaux (13, 14) ont des sections droites en forme de D.

3. Cathéter à canal double selon la revendication 1, qui comporte un embout conique creux (15) à l'extrémité distale dudit tube (10), la surface extérieure dudit embout conique (15) se confondant en solution de continuité avec la surface extérieure dudit tube (10), et la surface intérieure dudit embout conique (15) se confondant en solution de continuité avec la surface intérieure de l'un desdits canaux (14), l'extrémité distale de l'autre desdits canaux (13) étant écartée longitudinalement de l'extrémité distale dudit embout (15).

4. Cathéter à canal double selon la revendication 3, dans lequel ledit embout conique (15) constitue une connexion pleine entre l'extrémité distale dudit septum (12) et l'autre canal jusqu'à l'extrémité distale dudit embout (15) ; l'extrémité distale dudit élément de renforcement (50, 80) engageant ladite connexion pleine de telle sorte que des efforts appliqués à l'extrémité proximale dudit tube (10) sont transmis audit embout (15) par l'intermédiaire dudit élément de renforcement (50, 80).

5. Cathéter à canal double selon la revendication 3, dans lequel ledit septum (12) comporte une paire de nervures longitudinales espacées transversalement et formées comme partie intégrante dudit tube (10), ledit élément de renforcement (50) comportant une bande plate en un matériau rigide insérée longitudinalement entre lesdites nervures et collée à celui-ci.

6. Cathéter à canal double selon la revendication 5, dans lequel la dimension transversale de ladite bande de renforcement, dans la direction parallèle auxdites nervures, est supérieure au diamètre intérieur dudit tube (10), de telle sorte que ladite bande s'étende partiellement à l'intérieur des parois du tube (10).

7. Cathéter à canal double selon la revendication 1, dans lequel ledit élément de renforcement (80) comporte un élément tubulaire allongé enrobé dans la paroi de l'un desdits canaux (13, 14).

8. Cathéter à canal double selon la revendication 7, dans lequel à la fois un desdits canaux (13) et ledit élément de renforcement (80) ont des sections droites transversales en forme de D.

9. Cathéter à canal double selon la revendication 7, dans lequel les parois intérieures dudit élément de renforcement (80) ont un revêtement constitué par le même matériau (81) que celui qui constitue ledit tube (10), de telle sorte que ledit élément de renforcement tubulaire (80) est isolé des fluides et des tissus du corps.

10. Cathéter à canal double selon la revendication 1, dans lequel ledit élément de renforcement (50, 80) est constitué par du nylon.

11. Cathéter à canal double selon la revendication 1, dans lequel ledit élément de renforcement (50, 80) et une portion dudit tube à l'extrémité distale de celui-ci s'interpénètrent.

12. Cathéter à canal double selon l'une des revendications 1 à 11, dans lequel ladite extrémité distale dudit embout (50, 80) est raccordée à l'extrémité distale dudit tube (10) et comporte des ouvertures (51, 52, 53), des portions dudit tube (10) s'étendant dans une direction transversale à travers lesdites ouvertures (51, 52, 53) pour l'interpénétration de ladite bande (50, 80) et dudit tube (10), ledit élément de renforcement (50, 80) s'étendant sur la longueur dudit tube (10) et étant constitué par un matériau qui est essentiellement plus rigide que le matériau qui constitue ledit tube (10) de telle sorte que l'on peut faire avancer le cathéter longitudinalement lorsqu'on lui oppose une résistance, par l'application d'un effort proximal opposé à la résistance, de manière à ce que ledit canal (13, 14) ne s'affaisse pas par déformation dudit septum (12) lorsque la pression différentielle du système de dialyse s'établit à travers ledit septum (12).

13. Cathéter à canal double selon la revendication 1, comportant une spirale (70) résistant à la déformation en vrille, constitué par un matériau de renforcement bobiné sur une longueur substantielle dudit tube (10).

14. Cathéter à canal double selon la revendication 13, dans lequel ladite spirale (70) est enrobée dans la paroi cylindrique dudit tube (10).

15. Cathéter à canal double selon la revendication 3, dans lequel ledit embout conique (15) est moulé directement au-dessus de l'extrémité dudit tube (10).

16. Cathéter à canal double selon la revendication 3, dans lequel l'extrémité distale de l'un desdits canaux (13, 14) et ledit septum sont connectés audit embout conique (15) par un matériau plein.

17. Cathéter à canal double selon la revendication 3, dans lequel ledit embout conique (15) est préformé et collé à l'extrémité dudit tube (10), une portion dudit embout préformé (15) s'étendant à l'intérieur dudit tube (10) pour constituer la partie terminale de l'un desdits canaux (13).

18. Cathéter à canal double selon l'une des revendications 1 à 17, dans lequel au moins une ouverture (21, 22, 23, 24) ménagée dans la paroi latérale dudit tube aboutit à l'intérieur de l'autre desdits canaux (13).

19. Cathéter à canal multiple selon la revendication 18, dans lequel ledit élément de renforcement (50) peut être enlevé dudit tube (10), de telle sorte que l'élément de renforcement (80) peut être retiré dudit tube (10) après que le cathéter a été inséré dans le corps du patient.
